(19) Europäisches Patentamt
European Patent Office
Office européen des brevets

(11) Veröffentlichungsnummer: **0 337 929 A1**

(12) **EUROPÄISCHE PATENTANMELDUNG**

(21) Anmeldenummer: **89730087.7**

(22) Anmeldetag: **31.03.89**

(51) Int. Cl.[4]: **C 07 D 233/60**
A 61 K 31/415

(30) Priorität: **31.03.88 DE 3811574**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Strehlke, Peter, Dr.**
**Droysenstrasse 10A**
**D-1000 Berlin 12 (DE)**

**Bohlmann, Rolf, Dr.**
**Kühler Weg 6a**
**D-1000 Berlin 19 (DE)**

**Henderson, David, Dr.**
**Jahnstrasse 17**
**D-1000 Berlin 28 (DE)**

**Nishino, Yukishige, dr.**
**Lanzendorfer Weg 14**
**D-1000 Berlin 22 (DE)**

(54) **N-substituierte Imidazole, Verfahren zu ihrer Herstellung sowie ihre Verwendung in Arzneimitteln.**

(57) Die Erfindung betrifft N-substituierte Imidazole, der allgemeine Formel I

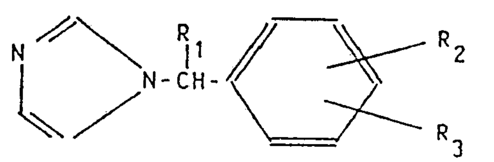

worin
$R_1$ ein Wasserstoffatom, einen gesättigten oder ungesättigten, gerad- oder verzweigtkettigen Kohlenwasserstoffrest oder einen cyclischen Kohlenwasserstoffrest oder einen Cycloalkylalkylrest oder einen Arylalkylrest,
$R_2$ eine gegebenenfalls substituierte Benzyloxygruppe,
$R_3$ eine Cyanogruppe, eine gegebenenfalls substitiuierte Alkanoylgruppe eine gegebenenfalls substituierte Benzoyl gruppe oder eine gegebenenfalls derivatisierte Carboxylgruppe bedeutet, sowie die Verbindungen in Form ihrer Salze, Verfahren zu ihrer Herstellung sowie ihre Verwendung in Arzneimitteln.

Die erfindungsgemäßen Verbindungen besitzen aromatasehemmende Eigenschaften und sind zur Therapie von östrogenbedingten Krankheiten geeignet.

EP 0 337 929 A1

**Beschreibung**

**N-substituierte Imidazole, Verfahren zu ihrer Herstellung sowie ihre Verwendung in Arzneimnitteln**

Die Erfindung betrifft N-substituierte Imidazole, Verfahren zu ihrer Herstellung sowie ihre Verwendung in Arzneimitteln.

Die erfindungsgemäßen Verbindungen werden durch die allgemeinen Formel I

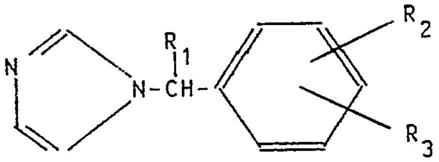

(I)

beschrieben,
worin
$R_1$ ein Wasserstoffatom, einen gesättigten oder ungesättigten, gerad- oder verzweigtkettigen Kohlenwasserstoffrest mit 1 bis 10 Kohlenstoffatomen oder einen cyclischen Kohlenwasserstoffrest mit 3 bis 9 Kohlenstoffatomen oder einen Cycloalkylalkylrest mit 4 bis 12 Kohlenstoffatomen oder einen Arylalkylrest mit 7 bis 12 Kohlenstoffatomen,
$R_2$ eine gegebenenfalls substituierte Benzyloxygruppe,
$R_3$ eine Cyanogruppe, eine gegebenenfalls substitiuierte Alkanoylgruppe mit 2 bis 10 Kohlenstoffatomen, eine gegebenenfalls substituierte Benzoyl gruppe oder eine gegebenenfalls derivatisierte Carboxylgruppe bedeutet.

Die Verbindungen der allgemeinen Formel I können auch in Form ihrer Salze vorliegen.

Die als Rest $R_1$ infrage kommenden gesättigten oder ungesättigten, gerad- oder verzweigtkettigen Kohlenwasserstoffreste mit 1 bis 10 Kohlenstoffatomen sind beispielsweise der Methyl-, Ethyl-, Propyl-, Propenyl-, Isopropyl-, Butyl-, Hexyl-, Octyl- und Decylrest, wobei der Propylrest bevorzugt ist. Als cyclische Kohlenwasserstoffreste mit 3 bis 9 Kohlenstoffatomen sind beispielsweise der Cyclopentyl- und Cyclohexylrest zu nennen. Als Cycloalkylalkylreste mit 4 bis 12 Kohlenstoffatomen sind beispielsweise der Cyclopentylmethyl- und Cyclohexylmethylrest zu nennen. Als Arylalkylrest mit 7 bis 10 Kohlenstoffatomen ist beispielsweise der Benzylrest zu nennen.

Die als Rest $R_2$ infrage kommenden gegebenenfalls substituierten Benzyloxygruppen können als Substituenten am Aromaten ein oder mehrere Halogenatome, $C_{1-4}$-Alkyl-, Hydroxyl-, Methoxy-, Amino- und Cyanogruppen enthalten. Als substituierte Benzyloxygruppen haben sich die 3-Brom-, 4-Brom-, 4-Chlor-, 2,3-, 2,4-, 4,5- und 4,6-Dichlorbenzyloxygruppen als besonders geeignet erwiesen.

Die als Rest $R_3$ infrage kommenden gegebenenfalls substituierten Alkanoylgruppen sind solche mit 2 bis 10 Kohlenstoffatomen. Beispielsweise kann diese Alkanoylgruppe gerad- und verzweigtkettig sein und an beliebigen Stellen der Kette ein- oder mehrfach substituiert sein.

Als Substituenten der Alkanoylgruppe sind Halogenatome, Methoxy-, Amino-, Hydroxyl- und Cyanogruppen geeignet. Als bevorzugte Alkanoylgruppen sind Acetyl-, Propinoyl-, Butyryl-, Valeryl- und Caproylgruppen zu nennen. Als Substituenten der als Rest $R_3$ infrage kommenden substituierten Benzoylgruppe sind Halogenatome $C_{1-4}$-Alkyl-, Methoxy-, Amino-, Hydroxyl- und Cyanogruppen, wobei ein oder mehrere Substituenten an beliebigen Stellen der Benzoylgruppe stehen können, geeignet.

Weiter hat $R_3$ die Bedeutung einer gegebenenfalls derivatisierten Carboxylgruppe. wobei als Derivate beispielsweise Carbonsäureester und Carbonsäureamide zu nennen sind. Als besonders geeignet haben sich Methyl-, Ethyl- und Butylester sowie Carbonsäureamid, Carbonsäureisopropylamid, Carbonsäureanilid und Pyrrolidinylamid erwiesen.

Die Reste $R_2$ und $R_3$ können an allen infrage kommenden Positionen des Phenylringes stehen.

Als mögliche Salze der Verbindungen der allgemeinen Formel I sind physiologisch verträgliche Salze von organischen oder anorganischen Säuren zu nennen. Als Salze sind besonders geeignet das Malonat, das Succinat, das Hydrochlorid und das Hydrobromid.

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimulierten Tumoren, wie zum Beispiel Mammakarzinom oder Prostatahyperplasie (The Lancet, 1984, S. 1237-1239).

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulationen durch Östrogenentzug zu hemmen.

Wahrscheinlich sind Aromatasehemmer auch zur Behandlung des drohenden Herzinfarktes geeignet, da erhöhte Östrogenspiegel beim Mann einem Herzinfarkt vorangehen können (US-Patent 4.289.762).

Bekannte, aromatasehemmende Wirkung aufweisende Substanzen sind neben Steroiden auch nichtsteroidale Substanzen; wie die zum Beispiel in den europäischen Patentanmeldungen, Veröffentlichungsnummern

0165777 bis 0165784 beschriebenen diversen Stickstoffheterocyclen, die in J.Med.Chem. 1986, 29, Seiten 1362-1369 beschriebenen substituierten Glutarsäureimide, die in der europäischen Patentanmeldung mit der Veröffentlichungsnummer 0165904 beschriebenen substituierten Imidazopyridine und die in der europäischen Patentanmeldungmit der Veröffentlichungsnummer 0236940, beschriebenen substituierten heterocyclisch substituierten Toluolnitrile.

Die Verbindungen der vorliegenden Anmeldung zeichnen sich dadurch aus, daß sie das Enzymsystem der Aromatase selektiv hemmen und andere Enzymsysteme in nennenswerter Weise nicht beeinträchtigen.

Weiter betrifft die Erfindung die Verwendung von bereits bekannten Verbindungen der Formel Ia (siehe Anspruch 4) mit $R_{3a}$ in der Bedeutung einer Nitro- oder Aminogruppe als Aromatasehemmer. Diese Verbindungen werden als antifungal wirksame Substanzen beschrieben in Eur. J. Med. Chem., 1979 (14), Seiten 231-237, und in der deutschen Offenlegungsschrift 24 18 502. Die aromatasehemmende Wirkung dieser bekannten Verbindungen ist dadurch ausgezeichnet, daß sie das Aromataseenzymsystem selektiv hemmen.

Als solche literaturbekannte Verbindungen, die die Aromatase in besonders selektiver Art und Weise hemmen, sind beispielsweise 4-Nitro-2-(1-(1-imidazolyl)-butyl)-phenyl)-(2,4-dichlorbenzyl)-ether, Hydrochlorid, 4-Amino-2-(1-(1-imidazolyl)-butyl)-phenyl)-(2,4-dichlorbenzyl)-ether, Hydrochlorid und (2,4-Dichlorbenzyl)-(2-(1-imidazolylmethyl)-4-nitro-phenyl)-ether zu nennen.

Mit dem nachfolgend beschriebenen biologischen Test werden die Enzymaktivitäten der Vergleichsverbindung 2,4-Dichlorbenzyl-{2-[1-(1-imidazolyl)-butyl]-phenyl}-ether (EP-A 0165 777) sowie der beiden erfindungsgemäßen Verbindungen 4-[1-(1-Imidazolyl-butyl]-3-(2,4-dichlorbenzyloxy)-benzonitril (Verbindung 1) und 3-(4-Brombenzyloxy)-4-[1-(1-imidazolyl)-butyl]-benzanilid (Verbindung 2) bestimmt.

Die Fähigkeiten der Verbindungen, das Enzymsystem der Aromatase zu inhibieren, wird an Microsomen, erhalten aus Humanplacenta, getestet. Die Freisetzung von Tritium-markiertem Wasser ($^3H_2O$), das als Reaktionsprodukt bei der Aromatisierung von [1β-$^3$H]-Androstendion zum Östron freigesetzt wird, wird nach der Methode von Thompson und Siieteri [J.Biol.Chem., 249, 5364-72 (1974)] gemessen. Die entsprechenden Hemmwerte ($K_1$, Aromatase) werden durch graphische Bestimmung, der Auftragung von 1/V gegen die Hemmkonzentration, gemäß der Methode von Dixon [Biochem.J. 94, 760 (1965)] bestimmt.

| | $K_i$ Aromatase |
|---|---|
| Vergleichsverbindung | 4,5 nmol/L |
| Verbindung 1 | 0,11 nmol/L |
| Verbindung 2 | 0,64 nmol/L |

Die zu verabreichende Menge der Verbindungen schwankt innerhalb eines weiten Bereichs und kann jede wirksame Menge abdecken. In Abhängigkeit des zu behandelnden Zustands und der Art der Verabreichung kann die Menge der verabreichten Verbindungen 0,0001-10 mg/kg Körpergewicht, vorzugsweise 0,001-1 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch vertraglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 0,05-50 mg des Wirkstoffes (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden zum Beispiel Öle mit oder ohne Zusatz eines Lösungsvermittlers, eines oberflächenaktiven Mittels, eines Suspendier- oder Emulgiergemisches verwendet. Als Beispiele für Öle seien Olivenöl, Erdnußöl, Baumwollöl, Sojabohnenöl, Rizinusöl und Sesamöl genannt.

Die Verbindungen lassen sich auch in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Applikation zum Beispiel in einem Pflaster eingearbeitet werden.

Die Erfindung betrifft somit auch pharmazeutische Präparate und die Verwendung der Verbindungen zur Herstellung dieser Präparate zur Behandlung von östrogenbedingten Krankheiten.

Die Erfindung betrifft ferner Verfahren zur Herstellung von substituierten Imidazolen der allgemeinen Formel I gemäß Anspruch 1, dadurch gekennzeichnet, daß man

a) eine Verbindung der allgemeinen Formel II

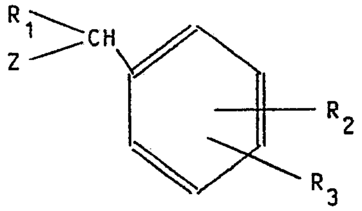

(II) ,

worin
$R_1$, $R_2$ und $R_3$ die in Formel I genannte Bedeutung haben, und
Z eine Fluchtgruppe bedeutet,
mit einer Verbindung der allgemeinen Formel III

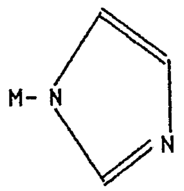

(III) ,

worin,
M ein Wasserstoffatom oder ein Alkalimetallatom bedeutet, oder
b) eine Verbindung der allgemeinen Formel IV

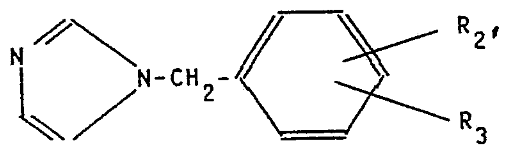

(IV) ,

worin
$R_{2'}$ dieselbe Bedeutung hat wie $R_2$ in Formel I mit der Maßgabe, daß $R_{2'}$ ein elektronenziehender, nicht deprotonierbarer Rest ist, sowie
$R_3$ die in Anspruch 1 genannte Bedeutung hat,
mit einer Verbindung der allgemeinen Formel V
$R_1$-Z (V),
worin
$R_1$ die in Formel I genannte Bedeutung hat und
Z eine Fluchtgruppe bedeutet, oder
c) eine Verbindung der allgemeinen Formel VI

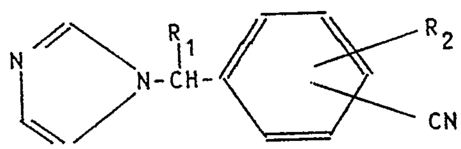

(VI) ,

worin
$R_1$ und $R_2$ die in Formel I genannte Bedeutung haben,
hydrolytisch in die Carboxylverbindung oder in das Carbonsäureamid, gegebenenfalls die Carboxylverbindung mit einem Alkohol zum Ester oder mit einem Halogenierungsmittel in das Carbonsäurehalogenid und mit Ammoniak oder Aminen in das Carbonsäureamid oder in die substituierten Carbonsäureamide, gegebenenfalls eine Verbindung der allgemeinen Formel VI mit einer metallorganischen Verbindung in das Alkanoyl- oder Benzoylderivat
umsetzt.

Für die unter a) genannten Umsetzungen sind als Fluchtgruppen Z literaturbekannte, leicht substituierbare Gruppen geeignet. Als solche zur Fluchtgruppenfähigkeit geeignete funktionelle Gruppen seien beispielsweise die Mesyl-, Tosyl-, Triflat- und Acetylgruppe genannt. Als geeignet haben sich auch die Halogene Chlor und Brom erwiesen. Die geeigneten Methoden sind u.a. in Eur.J.Med.Chem. 1979, Seiten 231-237 beschrieben.

Als Substituenten M der allgemeinen Formel III sind beispielsweise das Wasserstoffatom und Alkalimetallatome geeignet. Als Alkalimetallatome sind Lithium, Natrium und Kalium bevorzugt.

Die Reaktionen, die unter a) aufgeführt sind, die Umsetzungen von Verbindungen der allgemeinen Formel II mit Verbindungen der allgemeinen Formel III, lassen sich in allen inerten organischen Lösungsmitteln durchführen. Als Lösungsmittel kommen beispielsweise Dimethylformamid, Dimethylsulfoxyd und diverse Ether (zum Beispiel Tetrahydrofuran, Dioxan und Diethylether) infrage.

Die Herstellung der Verbindung der allgemeinen Formel I kann auch nach Verfahren b) erfolgen. Es werden Verbindungen der allgemeinen Formel IV mit Verbindungen der allgemeinen Formel V umgesetzt. Als Fluchtgruppe Z der Verbindungen der allgemeinen Formel V sind alle gängigen Fluchtgruppen, zum Beispiel die bei a) genannten, geeignet.

Zur Herstellung der Verbindungen der allgemeinen Formel I nach dem Verfahren b) wird mit Basen aus den Verbindungen der allgemeinen Formel IV das entsprechende Benzylanion hergestellt (der Rest $R_{2'}$ muß elektronenziehend und darf selbst nicht deprotonierbar sein) und dieses mittels Standardmethoden mit den

Verbindungen der allgemeinen Formel V umgesetzt. Die benzylische $-CH_2$-Gruppe in den Verbindungen der allgemeinen Formel IV läß sich mittels Basen, zum Beispiel durch Umsetzung mit tertiären Aminen, Natriumhydrid, Lithiumhydrid, Lithiumdiisopropylamid und Magnesiumhydrid deprotonieren und mit Verbindungen der allgemeinen Formel V zur Reaktion bringen.

Die erfindungsgegemäßen Verbindungen der allgemeinen Formel I lassen sich auch nach dem Verfahren c) herstellen. Dabei werden Verbindungen der allgemeinen Formel VI nach Standardmethoden umgesetzt.

Die Cyanoverbindungen lassen sich unter hydrolytischen Bedingungen in die Carbonsäureamide oder Carbonsäuren überführen.

Die erhaltenen Carboxylverbindungen lassen sich mit Alkoholen unter Standardbedingungen der Veresterung in die entsprechenden Carbonsäureester umsetzen.

Ebenso ist es möglich, Carbonsäureester über die Reaktionssequenz Carbonsäure und Carbonsäurehalogenid zu erhalten. Die als Zwischenprodukte erhältlichen Carbonsäurehalogenide können zur Herstellung von Carbonsäureamiden benutzt werden. Beispielsweise läßt sich ein Carbonsäurechlorid mit Ammoniak oder einem Amin zum Carbonsäureamid oder substituierten Carbonsäureamid umsetzen.

Es ist aber auch möglich, die Carbonsäureestergruppe unter Bedingungen einer Umesterungsreaktion mit Alkoholen in Gegenwart einer Säure, beispielsweise 4-Toluolsulfonsäure, in andere Ester umzuwandeln.

Die Herstellung der gegebenenfalls substituierten Alkanoyl- und Benzoylverbindungen der allgemeinen Formel I erfolgt durch Umsetzung von Verbindungen der allgemeinen Formel VI mit metallorganischen Verbindungen, beispielsweise Grignard-Reagenzien.

Beispiel 1

4-(2,4-Dichlorbenzyloxy)-3-[1-(1-imidazolyl)-butyl]-benzonitril

Durch Veresterung von 4-Hydroxybenzonitril mit Buttersäurechlorid wird 4-Butyryloxybenzonitril erhalten, das weiter durch Fries- Reaktion in Gegenwart von Aluminiumtrichlorid in Nitrobenzol in das 3-Butyryl-4-hyroxybenzonitril (Schmelzpunkt: 74-76 °C) umgesetzt wird. Die phenolische Hydroxylgruppe wird mit 2,4-Dichlorbenzylchlorid in Gegenwart von Natriumhydrid in Dimethylformamid zum 3-Butyryl-4-(2,4-dichlorbenzyloxy)-benzonitril (Schmelzpunkt: 79-80 °C) verethert. Durch Reduktion der aromatischen Ketogruppe mit Natriumborhydrid in wäßrigem Dioxan wird das 4-(2,4-Dichlorbenzyloxy)-3-(1-hydroxybutyl)-benzonitril (Schmelzpunkt 120-121 °C] erhalten. Durch Chlorierung des substituierten Benzylalkohols mit Thionylchlorid wird das 3-(1-Chlorbutyl)-4-(2,4-dichlorbenzyloxy)-benzonitril (hellgelbes Öl) erhalten. Durch Reaktion der letztgenannten Verbindung mit Imidazol in Gegenwart von Natriumhydrid in Dimethylformamid bei Raumtemperatur wird das 4-(2,4-Dichlorbenzyloxy)-3-[1-(1-imidazolyl)-butyl]-benzonitril nach Umkristallisation aus Ether/Hexan vom Schmelzpunkt 132-133 °C, erhalten.

Beispiel 2

4-(3-Brombenzyloxy)-3-[1-(1-imidazolyl)-butyl]-benzonitril

Diese Verbindung wird analog Beispiel 1 erhalten. Das 4-(3-Brombenzyloxy)-3-[1-(1-imidazolyl)-butyl]-benzonitril schmilzt bei 117-118 °C.

Beispiel 3

4-(4-Brombenzyloxy)-3-[1-(1-imidazolyl)-butyl]-benzonitril

Diese Verbindung wird analog Beispiel 1 erhalten. Schmelzpunkt 160-162 °C.

Beispiel 4

{4-(4-Brombenzyloxy)-3-[1-(1-imidazolyl)-butyl]-phenyl}-pentyl-keton

Durch Reaktion des Nitrils aus Beispiel 3 mit Pentylmagnesiumbromid in Tetrahydrofuran wird das {4-(4-Brombenzyloxy)-3-[1-(1-imidazolyl)-butyl]-phenyl}-pentyl-keton als gelbes Öl erhalten.

Beispiel 5

3-(2,4-Dichlorbenzyloxy)-4-[1-(1-imidazolyl)-butyl]-benzonitril

Durch Veresterung von 3-Bromphenol mit Buttersäurechlorid wird 3-Brom-1-butyryloxybenzol erhalten, das weiter durch Fries-Reaktion und Veretherung der phenolischen Hydroxylgruppe mit 2,4-Dichlorbenzylchlorid in das 3-(2,4-Dichlorbenzyloxy)-4-butyryl-1-brombenzol umgesetzt wird. Durch Reaktion des substituierten Brombenzols mit Kupfer-(I)-cyanid in N-Methylpyrrolidon (3 Stunden bei 180 °C) wird das 3-(2,4-Dichlorbenzyloxy)-4-butyrylbenzonitril mit Schmelzpunkt 97-98 °C erhalten. Die aromatische Ketogruppe wird mit Natriumborhydrid in wäßrigem Dioxan zum 3-(2,4-Dichlorbenzyloxy)-4-(1-hydroxybutyl)-benzonitril, Schmelzpunkt 101-105 °C, reduziert. Durch Umsetzung mit Thionylchlorid wird 3-(2,4-Dichlorbenzyloxy)-4-(1-chlorbutyl)-benzonitril als gelbes Öl erhalten. Das substituierte Benzylchlorid wird mit dem Natriumsalz des Imidazols in Dimethylformamid zum 3-(2,4-Dichlorbenzyloxy)-4-[1-(imidazolyl)-butyl]-benzonitril (Schmelzpunkt 105-107 °C) umgesetzt.

Beispiel 6

3-(4-Brombenzyloxy)-4-[1-(1-imidazolyl)-butyl]-benzonitril
Diese Verbindung wird analog Beispiel 5 erhalten, Schmelzpunkt 107-110 °C.

Beispiel 7

3-(3-Brombenzyloxy)-4-[1-(1-imidazolyl)-butyl]-benzonitril, Hydrochlorid .
Diese Verbindung wird analog Beispiel 5 und anschließende Überführung der Base in das Hydrochlorid unter Standardbedingungen erhalten, Schmelzpunkt 158-162 °C.

Beispiel 8

4-(4-Brombenzyloxy)-3-[1-(1-imidazolyl)-butyl]-benzoesäure
1,5 g 4-(4-Brombenzyloxy)-3-[1-(1-imidazolyl)-butyl]-benzonitril (aus Beispiel 3) werden mit 3 g Kaliumhydroxid in einer wäßrigen Methanollösung 24 Stunden unter Rückflußsieden umgesetzt. Nach Aufarbeitung werden 1,1 g 4- (4-Brombenzyloxy)-3-[1-(1-imidazolyl)-butyl]-benzoesäure mit Schmelzpunkt 187-189 °C erhalten.

Beispiel 9

4-(4-Brombenzyloxy)-3-[1-(1-imidazolyl)-butyl]-benzanilid
Die substituierte Benzoesäure aus Beispiel 8 wird mit Thionylchlorid in das Säurechlorid und weiter mit Anilin in einem Dioxan/Tetrahydrofuran-Gemisch in das 4-(4-Brombenzyloxy)-3-[1-(1-imidazolyl)-butyl]-benzanilid mit Schmelzpunkt 159-166 °C umgesetzt.

Beispiel 10

3-(4-Brombenzyloxy)-4-[1-(1-imidazolyl)-butyl]-benzoesäure
Das Benzonitril aus Beispiel 6 wird analog Beispiel 8 zur 3-(4-Brombenzyloxy)-4-[1-(1-imidazolyl)-butyl]-benzoesäure mit Schmelzpunkt 155-158 °C umgesetzt.

Beispiel 11

3-(4-Brombenzyloxy)-4-[1-(1-imidazolyl)-butyl]-benzanilid
Die substituierte Benzoesäure aus Beispiel 10 wird analog Beispiel 9 in 3-(4-Brombenzyloxy)-4-[1-(1-imidazolyl)-butyl]-benzanilid mit Schmelzpunkt 168 °C umgesetzt.

Beispiel 12

{3-(4-Brombenzyloxy)-4-[1-(1-imidazolyl)-butyl]-phenyl}-pentyl-keton
Das substituierte Benzonitril aus Beispiel 6 wird analog Beispiel 4 mit Pentylmagnesiumbromid zum {3-(4-Brombenzyloxy)-4-[1-(1-imidazolyl)-butyl]-phenyl}-pentyl-keton, Schmelzpunkt 70-72 °C, umgesetzt.

Beispiel 13

3-(2,4-Dichlorbenzyloxy)-4-[1-(1-imidazolyl)-butyl]-benzoesäure
Das substituierte Benzonitril aus Beispiel 5 wird analog Beispiel 8 in die 3-(2,4-Dichlorbenzyloxy)-4-[1-(1-imidazolyl)-butyl]-benzoesäure umgesetzt.

Beispiel 14

3-(2,4-Dichlorbenzyloxy)-4-[1-(1-imidazolyl)-butyl]-benzoesäuremethylester
Die substituierte Benzoesäure aus Beispiel 13 wird mit Thionylchlorid in das entsprechende Säurechlorid und weiter mit Methanol in Gegenwart von Pyridin in den 3-(2,4-Dichlorbenzyloxy)-4-[1-(1-imidazolyl)-butyl]-benzoesäuremethylester mit Schmelzpunkt 104 °C umgesetzt.

**Patentansprüche**

1.) N-substituierte Imidazole der allgemeinen Formel I

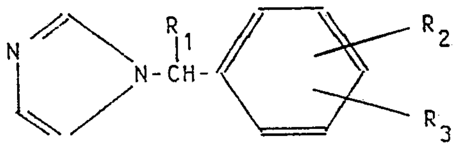

(I),

worin
$R_1$ ein Wasserstoffatom, einen gesättigten oder ungesättigten, gerad- oder verzweigtkettigen Kohlenwasserstoffrest mit 1 bis 10 Kohlenstoffatomen oder einen cyclischen Kohlenwasserstoffrest mit 3 bis 9 Kohlenstoffatomen oder einen Cycloalkylalkylrest mit 4 bis 12 Kohlenstoffatomen oder einen Arylalkylrest mit 7 bis 12 Kohlenstoffatomen,
$R_2$ eine gegebenenfalls substituierte Benzyloxygruppe,
$R_3$ eine Cyanogruppe, eine gegebenenfalls substituierte Alkanoylgruppe mit 2 bis 10 Kohlenstoffatomen, eine gegebenenfalls substituierte Benzoylgruppe oder eine gegebenenfalls derivatisierte Carboxylgruppe bedeutet,
sowie die Verbindungen in Form ihrer Salze.

2.) Verbindungen gemäß Anspruch 1:
4-(2,4-Dichlorbenzyloxy)-3-[1-(1-imidazolyl)-butyl]-benzonitril
{4-(4-Brombenzyloxy)-3-[1-(1-imidazolyl)-butyl]-phenyl}-pentyl-keton
4-(4-Brombenzyloxy)-3-[1-(1-imidazolyl)-butyl]-benzanilid
4-(4-Brombenzyloxy)-3-[1-(1-imidazolyl)-butyl]-benzoesäure
3-(2,4-Dichlorbenzyloxy)-4-[1-(1-imidazolyl)-butyl-benzonitril
3-(2,4-Dichlorbenzyloxy)-4-[1-(1-imidazolyl)-butyl]-benzoesäuremethylester
3-(2,4-Dichlorbenzyloxy)-4-[1-(1-imidazolyl)-butyl]-benzoesäure
3-(3-Brombenzyloxy)-4-[1-(1-imidazolyl)-butyl]-benzonitril, Hydrochlorid
4-(3-Brombenzyloxy)-3-[1-(1-imidazolyl)-butyl]-benzonitril
3-(4-Brombenzyloxy)-4-[1-(1-imidazolyl)-butyl]-benzoesäure
3-(4-Brombenzyloxy)-4-[1-(1-imidazolyl)-butyl]-benzanilid
{3-(4-Brombenzyloxy)-4-[1-(1-imidazolyl)-butyl]-phenyl}-pentyl-keton
4-(4-Brombenzyloxy)-3-[1-(1-imidazolyl)-butyl]-benzonitril
3-(4-Brombenzyloxy)-4-[1-(1-imidazolyl)-butyl]-benzonitril.

3.) Pharmazeutische Präparate, gekennzeichnet durch einen Gehalt an mindestens einer Verbindung gemäß Anspruch 1 oder 2.

4.) Verwendung der Verbindungen der allgemeinen Formel I a

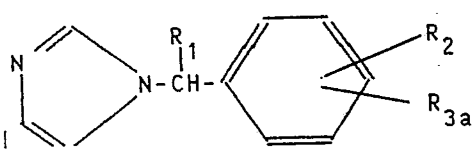

(Ia),

worin
$R_1$ und $R_2$ die in der allgemeinen Formel I genannte Bedeutung haben und
$R_{3a}$ eine Nitrogruppe oder eine Aminogruppe
bedeutet,
sowie die Verbindungen in Form ihrer Salze, zur Herstellung von Präparaten zur Behandlung von östrogen-bedingten Krankheiten.

5.) Verwendung von Verbindungen gemäß Anspruch 1 oder 2 zur Herstellung von Präparaten zur Behandlung von östrogen-bedingten Krankheiten.

6.) Verwendung von 4-Nitro-2-[1-(1-imidazolyl)-butyl]-phenyl-(2,4-dichlorbenzyl)-ether, Hydrochlorid, 4-Amino-2-[1-(1-imidazolyl)-butyl]-phenyl-(2,4-dichlorbenzyl)-ether, Hydrochlorid und (2,4-Dichlorbenzyl)-[2-(1-imidazolyl-methyl)-4-nitrophenyl]-ether zur Herstellung von Präparaten zur Behandlung von östrogen-bedingten Krankheiten.

7.) Verfahren zur Herstellung von substituierten Imidazolen der allgemeinen Formel I gemäß Anspruch 1, dadurch gekennzeichnet, daß man a) eine Verbindung der allgemeinen Formel II

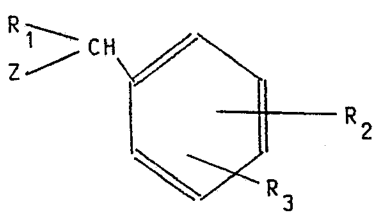

(II),

worin

$R_1$, $R_2$ und $R_3$ die in Anspruch 1 genannte Bedeutung haben, und
Z eine Fluchtgruppe bedeutet,
mit einer Verbindung der allgemeinen Formel III

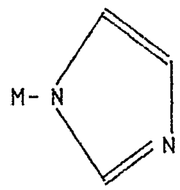

(III) ,

worin
M ein Wasserstoffatom oder ein Alkalimetallatom bedeutet, oder
b) eine Verbindung der allgemeinen Formel IV

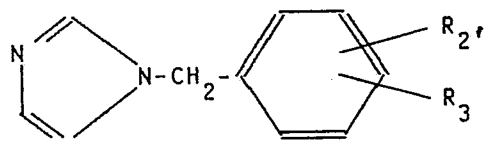

(IV) ,

worin
$R_{2'}$ dieselbe Bedeutung hat wie $R_2$ in Formel I, mit der Maßgabe, daß $R_{2'}$ ein elektronenziehender, nicht deprotonierbarer Rest ist, sowie
$R_3$ die in Formel I genannte Bedeutung hat,
mit einer Verbindung der allgemeinen Formel V
$R_1$-Z (V) ,
worin
$R_1$ die in Formel I genannte Bedeutung hat und
Z eine Fluchtgruppe bedeutet, oder
c) eine Verbindung der allgemeinen Formel VI

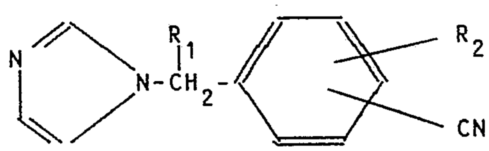

(VI) ,

worin
$R_1$ und $R_2$ die in Formel genannte Bedeutung haben,
hydrolytisch in die Carboxylverbindung oder in das Carbonsäureamid, gegebenenfalls die Carboxylverbindung mit einem Alkohol zum Ester oder mit einem Halogenierungsmittel in das Carbonsäurehalogenid und mit Ammoniak oder Aminen in das Carbonsäureamid oder in die substituierten Carbonsäureamide, gegebenenfalls eine Verbindung der allgemeinen Formel VI mit einer metallorganischen Verbindung in das Alkanoyl- oder Benzoylderivat
umsetzt.

Europäisches Patentamt

# EUROPÄISCHER RECHERCHENBERICHT

Nummer der Anmeldung

EP 89 73 0087

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 118 549 (SHIONOGI SEIYAKU K.K.) --- | | C 07 D 233/60<br>A 61 K 31/415 |
| A | GB-A-2 088 872 (SHIONOGI SEIYAKU K.K.) --- | | |
| A | GB-A-2 054 560 (SHIONOGI SEIYAKU K.K.) --- | | |
| A | GB-A-2 041 363 (PFIZER LTD) --- | | |
| A | CH-A- 648 844 (SIEGFRIED AG) --- | | |
| A | EP-A-0 106 060 (THE WELLCOME FOUNDATION LTD) --- | | |
| A | EP-A-0 162 359 (SHIONOGI & CO., LTD) --- | | |
| A | EP-A-0 142 754 (FERRER INTERMACIONAL S.A.) --- | | |
| A | DE-A-2 546 064 (SCHERING AG) --- | | |
| A,D | EP-A-0 236 940 (CIBA-GEIGY AG) ----- | | |

RECHERCHIERTE SACHGEBIETE (Int. Cl.4)

C 07 D 233/00
A 61 K 31/00

Der vorliegende Recherchenbericht wurde für alle Patentansprüche erstellt

| Recherchenort | Abschlußdatum der Recherche | Prüfer |
|---|---|---|
| DEN HAAG | 05-07-1989 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)